Europäisches Patentamt

European Patent Office

Office européen des brevets

(19)

(11) Publication number : **0 505 148 A1**

## (12) EUROPEAN PATENT APPLICATION

(21) Application number : **92302291.7**

(22) Date of filing : **17.03.92**

(51) Int. Cl.$^5$ : **C07K 15/06,** C07K 3/20, C07K 15/28, A61K 37/02, G01N 33/574

(30) Priority : **17.03.91 IL 97575**
**10.03.92 US 845581**

(43) Date of publication of application :
**23.09.92 Bulletin 92/39**

(84) Designated Contracting States :
**AT BE CH DE DK ES FR GB GR IT LI LU MC NL PT SE**

(71) Applicant : **YEDA RESEARCH AND DEVELOPMENT COMPANY LIMITED**
**P.O. Box 95**
**Rehovot 76100 (IL)**

(72) Inventor : **Yarden, Yosef, Dr.**
**7 Hachita St.**
**Rehovot (IL)**
Inventor : **Peles, Elior, Dr.**
**209 Ben Yehuda St.**
**Tel Aviv 63502 (IL)**

(74) Representative : **Brown, John David et al**
**FORRESTER & BOEHMERT**
**Franz-Joseph-Strasse 38**
**W-8000 München 40 (DE)**

(54) **Stimulating factor of the neu receptor.**

(57)    The *neu* proto-oncogene encodes a transmembrane glycoprotein related to the receptor for epidermal growth factor (EGF). Purification of a stimulating factor for the *neu* receptor to homogeneity from ras-transformed fibroblasts has now been achieved. Its identity as a *neu* receptor stimulating factor has been established by covalent crosslinking experiments. The factor stimulates the tyrosine phosphorylation of the *neu* receptor at low concentrations. The factor inhibits the proliferation and promotes the differentiation of human carcinoma cells.

EP 0 505 148 A1

Jouve, 18, rue Saint-Denis, 75001 PARIS

Field Of The Invention

This invention relates to a novel, purified mammalian proteinaceous factor which interacts with and stimulates the *neu* receptor and modulates cellular proliferation and differentiation.

Background Of The Invention

The enzymatic catalysis of the transfer of the phosphate group to tyrosyl residues of polypeptides, achieved by tyrosine kinase enzymes, appears to be associated with the control of cell growth. Several receptors for polypeptide growth factors belong to a family of transmembrane glycoproteins with intrinsic tyrosine kinase activity (Ullrich, A., and Schlessinger, J., *Cell* 61:203-212, 1990). This group, which includes the receptors for the epidermal growth factor (EGF), insulin, and the platelet derived growth factor (PDGF), is characterized by the presence of a single transmembrane domain which connects a large extracellular ligand-binding domain with a cytoplasmfacing protein kinase catalytic core. In addition to receptors for well-known growth and maintenance factors, several members of this family of tyrosine kinases appear to be regulated by yet unknown ligands (Hanks, S.K., *Cur. Op. Struct. Biol.* 1:369-383, 1991).

The protein encoded by the *neu* gene (also known as the c-*erb*B-2 or HER-2 gene) is an example of such a receptor-like tyrosine kinase. First identified by its amplification in a human mammary carcinoma (King, C.R. et al., *EMBO J.* 7:164-165, 1988) and by virtue of its relatedness to the EGF-receptor (Schechter, A. L. et al., *Nature* 312:513-516, 1984; Semba, K. et al., *Proc. Natl. Acad. Sci. USA* 82:6497-6501, 1985), the full-length gene codes for a transmembrane tyrosine kinase which shows extensive structural homologies with receptors for known growth factors, particularly the epidermal growth factor (EGF)-receptor (Coussens, L. et al., *Science* 230:1132-1139, 1985; Bargmann, C.I. et al., *Nature* 319:226-230, 1986; Yamamoto, T. et al., *Nature* 319:230-234, 1986). The protein is a 185 kDa transmembrane glycoprotein (i.e., p185$^{neu}$ protein) having an extracellular domain, a cytoplasmic portion which carries mostly tyrosine kinase sequences, and a relatively short transmembrane stretch. The rodent homolog of the *neu* gene has been shown to undergo activation as an oncogene by a point mutation within the transmembrane domain of amino acids (Bargmann, C.I. et al., *Cell* 45:649-657, 1986). The human gene is amplified in adenocarcinomas from several tissues, and the protein is overexpressed in approximately twenty-five percent of primary breast carcinomas (Kraus, M.H. et al., *EMBO J.* 6:605-610, 1987; Slamon, D.J. et al., *Science* 235:177-182, 1987; Varley, J.M. et al., *Oncogene* 1:423-430, 1987; Van de Vijver, et al., *Mol. Cell. Biol.* 7:2019-2023, 1987). An association between gene amplification and overexpression with clinical outcome has been reported in breast and ovarian cancers (Slamon, D.J. et al., *Science* 235:177-182, 1987; Varley, J.M. et al., *Oncogene* 1:423-430, 1987; Venter, D.J. et al., *Lancet* ii, 67-72, 1987; Zhou, D. et al., *Cancer Res.* 47:6123-6125, 1987; Berger, M.S. et al., *Cancer Cells* 48: 1238-1243, 1988; Tsuda, H. et al., *Cancer Res.* 49:3104-3108, 1989; Slamon, D.J. et al., *Science* 244:707-712, 1989). Consistent with these observations, ectopic overexpression of *neu* can transform rodent fibroblasts (Difiore, P.P. et al., *Science* 237:178-182, 1987: Hudziak, R.M. et al., *Proc. Natl. Acad. Sci. USA* 84:7159-7163, 1987).

By the use of monoclonal antibodies (Yarden, Y. *Proc. Natl. Acad. Sci. USA* 87: 2564-2573, 1987; Scott, G.K. et al., *J. Biol. Chem.* 266:14300-14305, 1991), and chimeric proteins (Lee, J. et al., *EMBO J.* 8:167-173, 1989; Lehvaslaiho, H. et al., *EMBO J.* 8:159-166, 1989; Peles, E. et al., *EMBO J.* 10:2077-2086, 1991; Fazioli, F. et al., *Mol. Cell Biol.* 11:2040-2048, 1991), it has been possible to demonstrate that the tyrosine kinase portion of the protein encoded by the *neu* gene can be stimulated to transmit growth regulatory biochemical signals. Based on the functional and structural similarities between the p185$^{neu}$ protein and known receptors for growth factors, these results led to the assumption that an endogenous ligand of p185$^{neu}$ protein exists. Using a series of biochemical assays to screen potential biological sources of the ligand, oncogene-transformed Rat1-EJ fibroblasts (Land, H. et al., *Nature* 304: 596-602, 1983) have been identified as producers of an activity that specifically affects the functions of the *neu* protein, including receptor down-regulation, autophosphorylation on tyrosine residues, and mitogenic activity (Yarden, Y. and Weinberg, R.A., *Proc. Natl. Acad. Sci. USA* 86:3179-3188, 1989). Other laboratories have reported the existence of neu-specific activating factors partially purified from human breast cancer cells (Lupu, R. et al., *Science* 249: 1552-1555, 1990), bovine kidney cells, (Huang, S.S. et al., *J. Biol. Chem.* 265:3340-3346, 1990), transformed human T-cells (Dobashi, K. et al., *Proc. Natl. Acad. Sci. USA* 88:8582-8586, 1991: Davis, J.G. et al., *Biochem Biophys. Res. Commun.* 179:1536-1542, 1991; *WO* 91/15230) and activated macrophages (Tarakhovsky, A. et al., *Oncogene* 6:2187-2196, 1991).

Summary Of The Invention

The present invention relates to mammalian proteinaceous stimulating factors for p185$^{neu}$ protein which are obtainable from media conditioned by *ras*-transformed fibroblasts.

In accordance with this invention, a specific stimulating factor (hereinafter the "*neu* receptor stimulating factor") for p185*neu* protein (hereinafter the "*neu* receptor") has now been purified to homogeneity from tissue culture media conditioned by *ras*-transformed rat fibroblasts, and various biochemical and biological properties of the ligand have been determined. Thus, the present invention provides a specific, purified mammalian stimulating factor for the *neu* receptor, obtainable in an essentially homogeneous form from media conditioned by *ras*-transformed fibroblasts. The *neu* stimulating factor is obtainable in accordance with this invention from such conditioned media by the combined use of at least three of the following four steps: heparin affinity chromatography, hydrophobic interaction chromatography, cation exchange chromatography and metal chelate affinity chromatography, and preferably with use of all four steps in the above order.

It has been found that the *neu* receptor stimulating factor of this invention is a glycoprotein with a molecular weight of about 44,000 daltons, (i.e., 44,000 ± 5,000). The stimulatory factor may or may not bind directly to the *neu* receptor as a ligand.

The stimulating factor is active at the picomolar range of concentration in stimulating *neu* receptor tyrosine phosphorylation, and yields amino acid sequences that show no homology with any known proteins. When tested on cultured human breast carcinoma cells, as is shown in the accompanying data, the stimulating factor induced phenotypic cellular differentiation that included markedly altered cellular morphology, and synthesis of milk components (i.e., casein and lipids). These changes were accompanied by an increase in nuclear area, an inhibition of cell growth, and the induction of DNA polyploidy.

On the basis of this and other biological evidence, including an analogy to growth and differentiation factors, it is envisaged that the *neu* receptor stimulating factor of this invention will have various potential applications. For one, the stimulating factor will most likely modulate proliferation and differentiation of cells expressing *neu* receptor and may accordingly be used for the stimulation of tissue regeneration after injury. Furthermore, the stimulating factor of this invention may be used as an anti-cancer drug either by itself (because of its activity as a differentiation factor), or to direct conjugated toxins, radionuclides or cytokines to cancer cells in certain cases, e.g., adenocarcinomas of the breast, ovary, stomach, or prostate gland that overexpress the *neu* receptor (because of its selective affinity for the *neu* receptor, similar to disease targeting of a toxin by conjugation to monoclonal antibodies).

Antibodies against the *neu* receptor stimulatory factor may be raised and used for certain diagnostic applications, e.g., measuring altered levels of stimulating factor in human diseases such as cancer, and may also serve to block the effects of the stimulating factor on cells, such as inhibition of cellular proliferation.

Briefly, this invention encompasses in its various aspects a purified mammalian receptor stimulating factor having the properties described herein, and which is obtained by the described method of purification; analogs, variants and active fragments thereof having substantially the same biological activity of the factor as can be assayed by the methods described herein (e.g. the *neu* Receptor Tyrosine Phosphorylation assay); methods of modulating cellular biological activity, e.g., proliferation and differentiation, by application of the factor alone or in conjunction with other therapeutic agents; antibodies raised against the factor which themselves have therapeutic value; methods of diagnosing abnormal levels of the *neu* receptor in the body, e.g., as overexpressed on the surface of tumor cells, by use of the factor and its binding properties; and methods of diagnosing abnormal levels of the factor itself by use of the anti-factor antibodies.

The invention is specifically illustrated in the Examples with reference to mammalian *neu* receptor stimulating factor purified from transformed rat fibroblasts. This factor is highly active in stimulating human *neu* receptor and in inducing growth arrest and phenotypic differences of human breast carcinoma cells, as shown. Because of these effects as well as the very substantial homology between similar factors of different mammalian species, it is expected that homologous *neu* receptor stimulating factors purified from human or other mammalian sources will induce substantially the same biological effects on human cells expressing *neu* receptor and have the same practical applications. Moreover, the described procedures will enable the purification of mammalian *neu* receptor stimulating factor from the conditioned media of the cell lines of other mammalian species that produce *neu* receptor stimulatory activity, e.g., MDA-MB-231 human breast carcinoma cells (Lupu, R. et al., supra); transformed human T cells (Dobashi, K. et al. supra), etc., and all these mammalian factors, including the human factor, are encompassed by the present invention.

Brief Description Of The Drawings

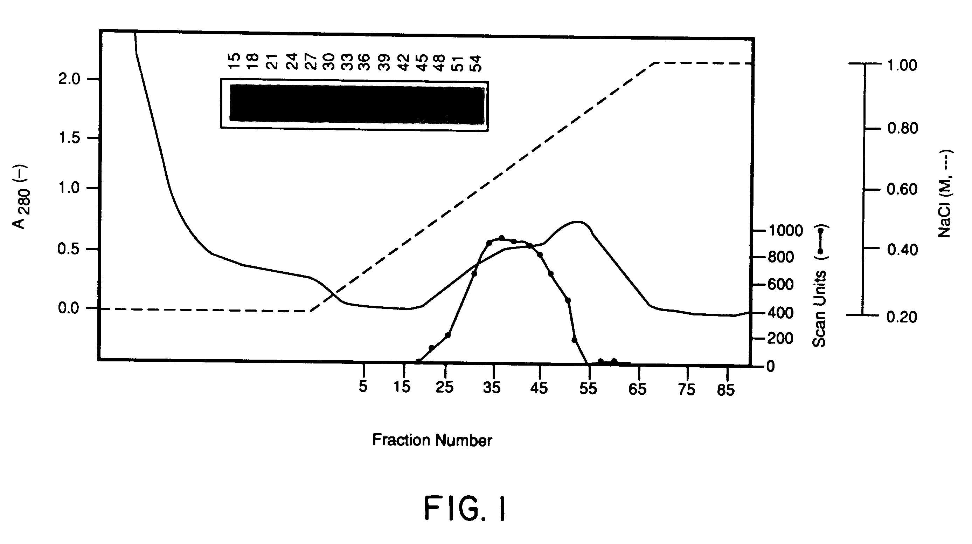

Figure 1 depicts initial fractionation of Rat1-EJ 1-1 cell conditioned media by Heparin-Sepharose column chromatography and shows the effect of the column fractions on tyrosine phosphorylation of the *neu* receptor in the tumor cell line MDA-MB-453. Serum-free medium was harvested from roller bottles containing Rat1-EJ 1-1 fibroblasts, concentrated, and loaded onto a Heparin-Sepharose column. After washing with PBS/0.2 M NaC1, the bound proteins were eluted with a gradient of salt (indicated by a dashed line). The elution phase of this chromatography step is depicted in the absorption profile. Samples of the fractions were tested for the presence of the *neu* receptor stimulating factor. Each protein sample was incubated with a monolayer of MDA-MB-453 cells. The cells were lysed and the whole cell lysates were Western blotted with antibodies to phosphotyrosine. The results are shown as an autoradiogram and the fraction numbers are indicated (inset). For quantitative determination of the stimulating factor, the autoradiogram was scanned by an automated scanning densitometer. The points on the graph of the scan corresponding to the fraction numbers in the autoradiogram are indicated by closed circles.

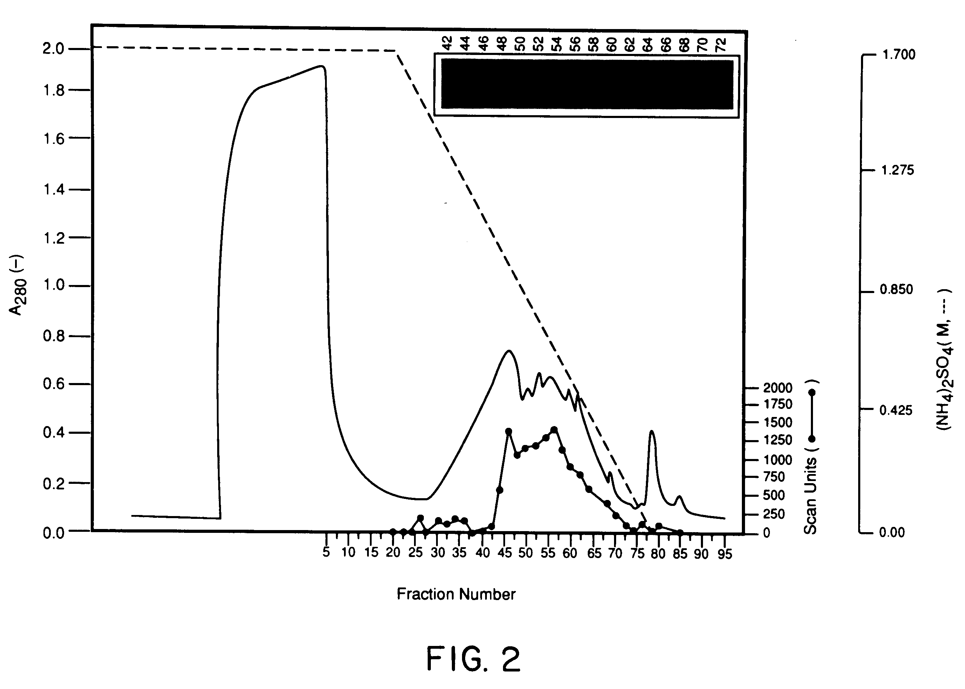

Figure 2 depicts additional purification of the *neu* ligand using Phenyl-Superose column chromatography. Proteins from the Heparin-Sepharose fractions with *neu* receptor stimulating activity (Fig. 1) were pooled, concentrated, and $(NH_4)_2SO_4$ was added to a final concentration of 1.7 M. This material was loaded onto a Phenyl-Superose column, and eluted from the column by decreasing the ammonium sulfate concentration. Fractions were tested for the presence of stimulating factor in the same manner as described for Fig. 1 (inset).

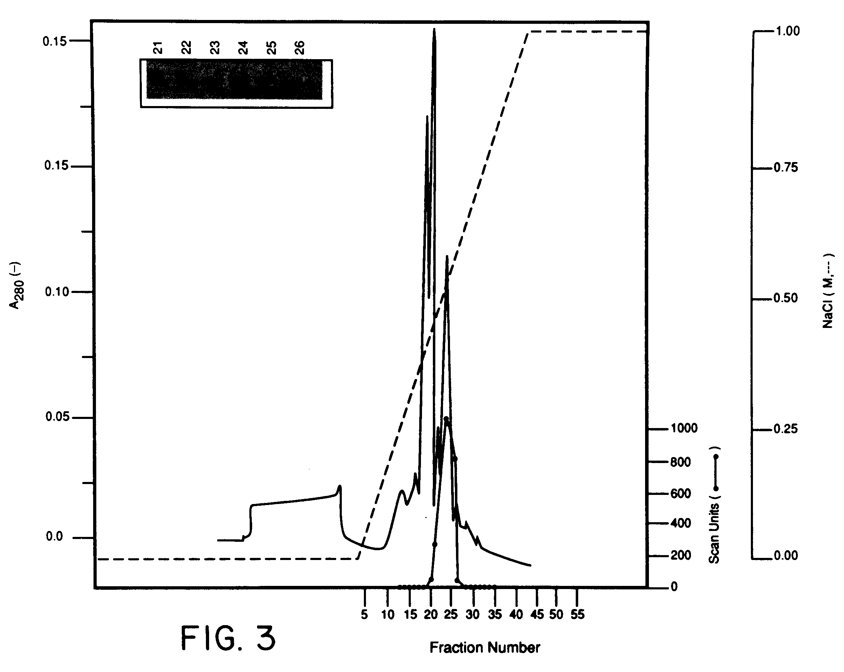

Figure 3 depicts further purification of the *neu* receptor stimulating factor using Mono S column chromatography. Proteins from the active Phenyl-Superose fractions of Fig. 2 were loaded into a Mono S cation exchange column, and eluted with a gradient of increasing salt concentration. Samples from individual column fractions were tested on MDA-MB-453 cells as in Figs. 1 and 2 for the induction of tyrosine phosphorylation of *neu* receptor (inset).

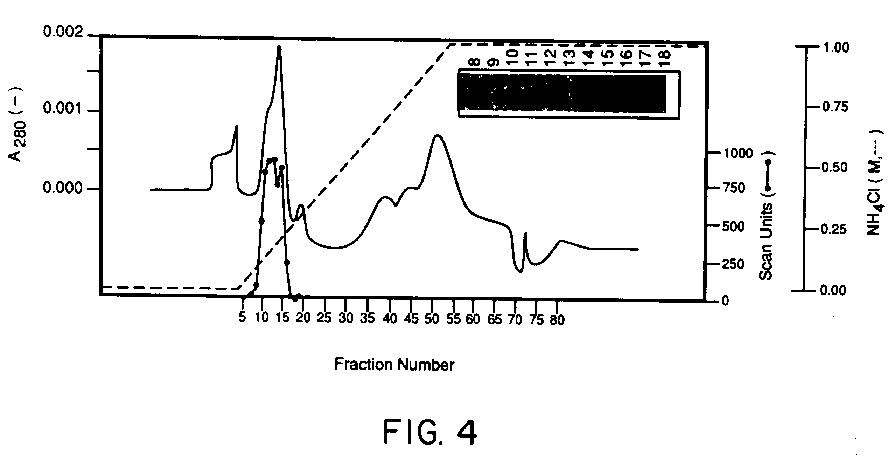

Figure 4 depicts continued purification of the *neu* ligand by metal chelate affinity chromatography. Proteins from active cation exchange fractions of Fig. 3 were fractionated by chromatography on a $Cu^{2+}$ chelating Sepharose column. Bound proteins were eluted with a gradient of increasing ammonium chloride and the fractions were tested for induction of tyrosine phosphorylation of *neu* receptor in the same manner as described above.

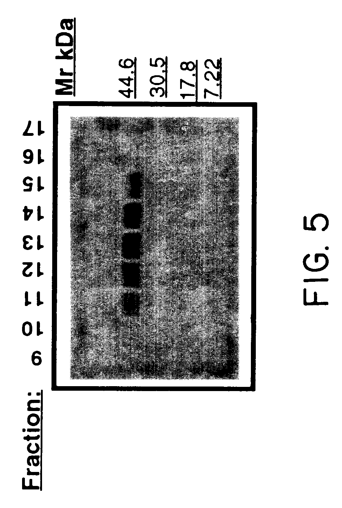

Figure 5 shows sodium dodecyl sulfate polyacrylamide gel electrophoresis (SDS-PAGE) analysis of proteins in active fractions from the chelating Sepharose column of Figure 4. Aliquots (0.01 ml) of the active fractions were subjected to electrophoresis in a 4-20% gradient polyacrylamide gel under reducing conditions. The gel was stained using a silver staining kit. The locations of the molecular weight marker proteins (BRL, Bethesda, MD) are indicated in kilodaltons.

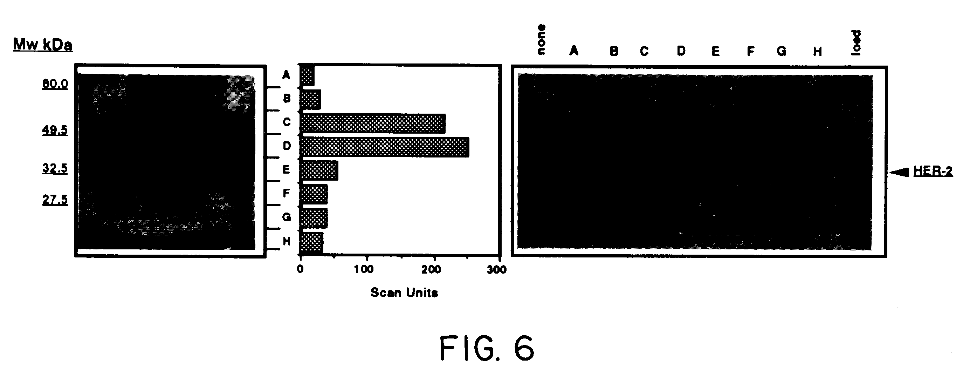

Figure 6 shows recovery of the *neu* receptor stimulating factor activity after gel electrophoresis. Two 100-nanogram samples of the stimulating factor purified through the four-step protocol referred to in Figs. 1-4 were separated by electrophoresis under non-reducing conditions in a 4-20% polyacrylamide gel. One lane was stained using a silver staining kit (left panel). The other was cut into eight strips (labeled A-H), and the proteins were electroeluted and tested on living MDA-MB-453 cells, by Western blotting, for the induction of *neu* receptor tyrosine phosphorylation. An autoradiogram of the Western blot is shown (right panel) and the location of the tyrosine phosphorylated *neu* receptor is indicated by an arrow ("none"-no protein added; "load" - 100 nanograms of purified stimulating factor without electrophoresis). Densitometric scanning of the Western blot is given in the center panel, with the eight bars (A-H) corresponding to stimulating factor activity eluted from each of the eight gel strips.

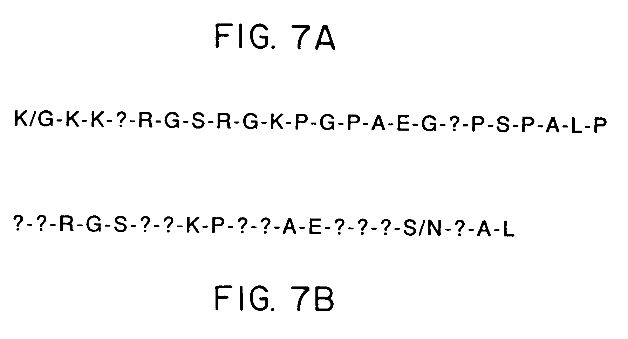

Figure 7 shows amino-terminal sequences of the *neu* receptor stimulating factor of this invention isolated using the purification protocol of Figs. 1-4. The primary sequence thus obtained is shown in the upper row (7A). A minor N-terminal variant, shown in the lower row (7B), was also detected. Unassigned positions are indicated by "?".

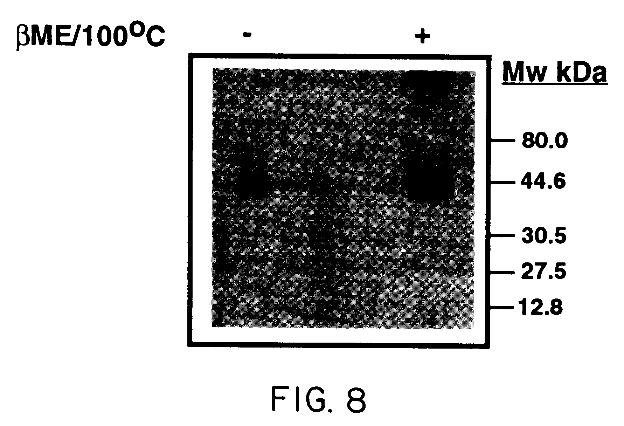

Figure 8 shows the *neu* receptor stimulating factor of this invention, under reducing and non-reducing conditions, after electrophoresis in a gradient SDS-PAGE gel. Prior to electrophoresis the 50 ng samples were either treated with β-mercaptoethanol reducing agent (2% vol.:vol.) at 100°C, or separated without a prior treatment, as indicated by "+" and "-", respectively. The silver stained gel is shown with the locations of the molecular weight marker proteins (BRL, Bethesda, MD).

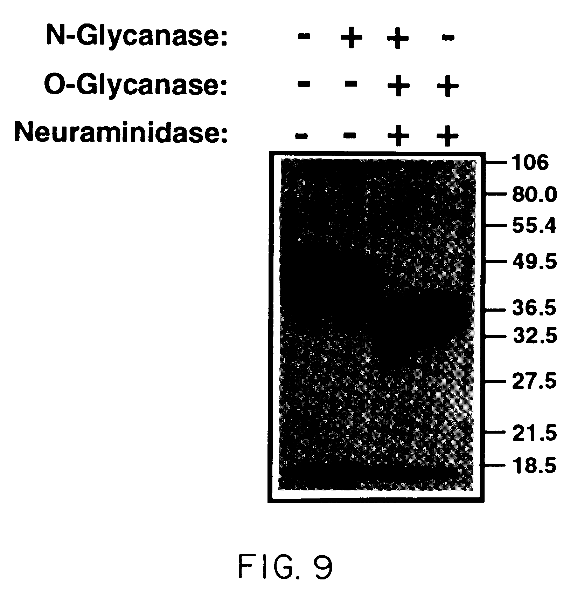

Figure 9 is an autoradiogram showing the effect of deglycosylation on the *neu* receptor stimulating factor of this invention. Radiolabelled stimulating factor was incubated with N-glycanase, O-glycanase and/or neuraminidase. After eighteen hours the reactions were stopped by heating to 95°C in SDS-PAGE gel sample buffer. The samples were then subjected to SDS gel electrophoresis (12% acrylamide), followed by exposure of the dried gel to film for five hours. Locations of molecular weight marker proteins are indicated by bars and kilodaltons.

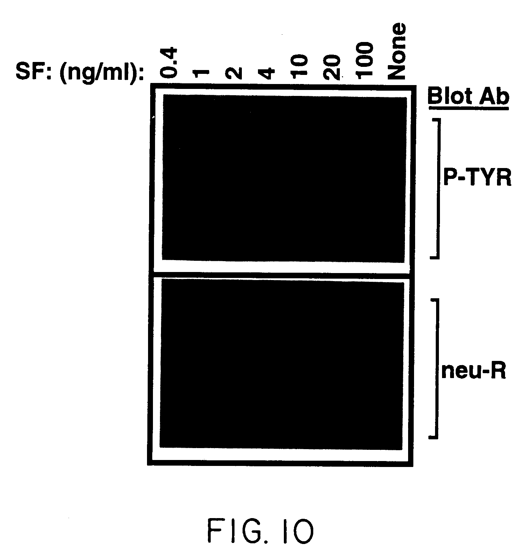

Figure 10 shows that tyrosine phosphorylation is stimulated by picomolar concentrations of the purified *neu* receptor stimulating factor. MDA-MB-453 cells were treated at 37°C with the indicated concentrations of the stimulating factor ("SF") purified through the four-step protocol described above. Cell lysates were prepared and subjected to Western blot analyses. The upper panel shows *neu* receptor phosphorylation detected with antibodies to phosphotyrosine (indicated as "p-TYR"). In the lower panel, the Western blot was stripped of antibodies bound to phosphotyrosine and reprobed with antibodies to the *neu* receptor ("*neu*-R"). It shows that the increased tyrosine phosphorylation is induced by increasing amounts of stimulating factor, and is not due to variable amounts of *neu* receptor in the cell lysates.

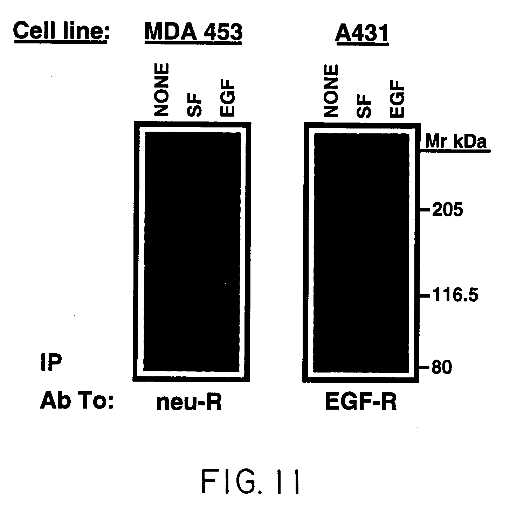

Figure 11 shows that the *neu* stimulating factor("SF") stimulates tyrosine phosphorylation of *neu* receptor, but not EGF-receptor, using two different carcinoma cell lines (MDA-MB-453 and A-431). The indicated cells were treated with either 10 ng/ml of stimulating factor or with 50 ng/ml of EGF. Immunoprecipitation with antibodies to *neu* receptor ("*neu*-R") or to EGF receptor ("EGF-R") occurred before gel electrophoresis and Western blotting with anti-phosphotyrosine antibodies.

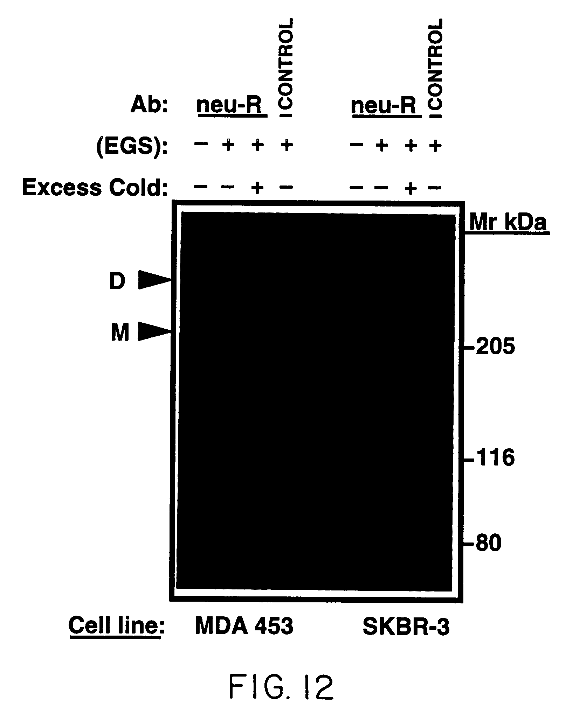

Figure 12 depicts an autoradiogram of electrophoresed (SDS-PAGE) complexes of *neu* receptor and radiolabelled *neu* receptor stimulating factor from two different human breast carcinoma cell lines (MDA-MB-453 and SK-BR-3). The complexes were chemically stabilized with a crosslinking reagent ("EGS"). The complexes were subjected to immunoprecipitation with either an anti-*neu* receptor ("*neu*-R") monoclonal antibody or a control antibody. Arrowheads indicate monomer (M) and dimer (D) forms. Formation of the complex was competed for by the addition of excess unlabelled stimulating factor ("Excess Cold"). Locations of the molecular weight markers are shown.

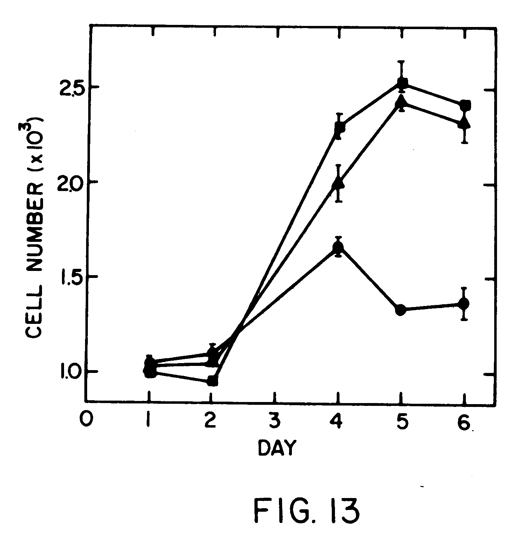

Figure 13 illustrates the growth inhibitory effects the stimulating factor on the MDA-MB-453 human breast carcinoma cell line. MDA-MB-453 cells were inoculated into multi-well culture dishes and after 24 hours their culture medium was replaced with serum-free medium. This was supplemented with 5 ng/ml of EGF (squares) or 5 ng/ml of stimulating factor (circles). Control cultures (triangles) received no EGF or *neu* receptor stimulating factor. The dishes were then incubated at 37°C and on the indicated days cell numbers were determined in duplicate cultures. The averages and their ranges (bars) are shown.

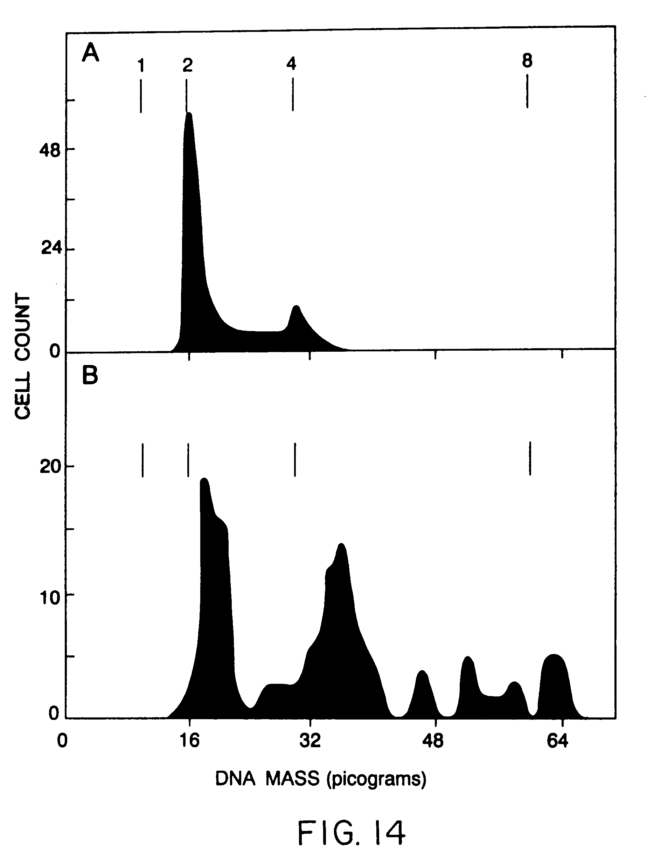

Figure 14 shows the distribution of DNA content in AU-565 carcinoma cells treated with the *neu* receptor stimulating factor of this invention. Cultures of the cells were treated with 6 ng/ml of the stimulating factor for five days. A control culture was left untreated. DNA content in individual cells was then determined by using the Feulgen reaction. Quantitation of the amount of DNA per cell was performed by computerized image analysis with a CAS-200 Image Analyzer (Bacus, S. et al., *Molecular Carcinogenesis* 3:350-362, 1990). The numbers at the top represent the DNA index, which was calculated by dividing the DNA content of each cell by the content of a reference human diploid cell at the $G_0/G_1$ stage of the cell cycle. The patterns are representative of at least twelve arbitrarily selected microscope fields (40X magnification). The top panel (A) represents control (untreated) cells; the bottom panel (B) represents *neu* receptor stimulating factor-treated cells.

## Detailed Description Of The Invention

In the following description, the invention will be illustrated with reference to specific methods and materials for testing samples for the presence of a stimulating factor of the *neu* receptor, and for the isolation and characterization of the stimulating factor.

## Antibodies

A monoclonal antibody to the kinase domain of *neu* receptor (Ab3) was obtained from oncogene Science (Uniondale, New York). A polyclonal rabbit antibody to the *neu* receptor carboxy terminus was raised as described in Peles, E. et al., *EMBO J.* 10:2077-2086 (1991). THE MOPC-141 plasmacytoma antibody was obtained from Sigma (St. Louis, MO). Rabbit antibodies to phosphotyrosine were prepared and affinity purified as described in Kamps, M.D. and Sefton, B.M., *Oncogene* 2: 305-316 (1988). A mouse monoclonal antibody that recognizes human β and χ casein was obtained from R.C. Coombs, Charing Cross Medical School, London, England.

## Maintenance and Characterization of Cell Lines

The Rat1-EJ 1-1 cell line (ATCC____) was generated by transfection of the human EJ gene (an activated Harvey *ras* gene) into Rat1 fibroblasts as described in the literature (Land, H. et al., *Nature* 304:596-602, 1983). After screening six independent, cloned Rat1-EJ cell lines for production of *neu* receptor stimulating factor using the *neu* receptor tyrosine phosphorylation assay described below, the Ratl-EJ 1-1 cell line was selected for large-scale conditioned media production. AU-565 cells were obtained from the Cell Culture Laboratory, Naval Biosciences Laboratory (Naval Supply Center, Oakland, CA). MCF7, SK-BR-3, A-431 and MDA-MB-453 cells were obtained from the American Type Culture Collection (Rockville, MD). The cell lines were cultured in RPMI-1640 or DME medium (GIBCO, Grand Island, NY) supplemented with 10% fetal bovine serum (Hyclone, Logan, Utah) in a humidified incubator with 5% $CO_2$ in air at 37°C.

Conditioned Medium

For large-scale production of conditioned medium, Rat1-EJ 1-1 cells were cultured first in T-flasks, expanded in 2-liter cell factories (Nunc), and then transferred into roller bottles. After reaching 80% confluency, the monolayers were incubated for eight hours at 37°C with serum-free medium to release cell-bound proteins. The medium was then replaced with fresh serum-free medium that was incubated with the cells for forty-eight hours at 37°C. A second forty-eight hour harvest of conditioned medium was also collected and pooled with the initial harvest.

*neu* Receptor Tyrosine Phosphorylation Assay

Samples were tested for *neu* receptor stimulating factor by assaying for induction of *neu* receptor tyrosine phosphorylation in living MDA-MB-453 human breast cancer cells. These cells overexpress *neu* receptor but do not express the EGF receptor (Kraus, M H. et al., *EMBO J.* 6:605-610, 1987). Samples of column fractions were usually diluted in PBS containing bovine-serum albumin (BSA, 1 mg/ml). These were added to individual wells of a 48-well dish (Nunc, Denmark) that contained $3 \times 10^5$ MDA-MB-453 cells that had been incubated in DME medium containing 0.5% fetal bovine serum for twelve hours prior to the assay. Each well contained 0.15 ml of PBS. Following five minutes of incubation at 37°C with the samples, the medium was aspirated and the cells lysed directly in 0.07 ml hot SDS-PAGE sample buffer with β-mercaptoethanol. The solubilized lysates were then heated for 5 minutes at 95°C, and one-third of each sample was subjected to electrophoresis in 6% SDS polyacrylamide gels. The gel-separated proteins were electrophoretically transferred onto nitrocellulose membranes. The membranes were first saturated for one hour at 22°C with 1% low-fat dry milk. An affinity-purified rabbit antibody to phosphotyrosine was then added and the incubation continued for 1 hour. For detection, the filters were washed with TTBS solution (0.05% Tween-20 in 20 mM Tris-HC1, pH 7.6, and 17 mM NaC1) and reacted for forty-five minutes at 22°C with horseradish peroxidase-conjugated protein A. The enzyme was removed by four washes with TTBS, and the filters were reacted for 1 minute with a chemiluminescence reagent (ECL, Amersham). The filters were then exposed to a high performance luminescence detection film (Hyperfilm-ECL, Amersham) for thirty seconds to thirty minutes. For quantitation of the signals, parallel assays were performed with no stimulation of the cells or with a hypertonic stimulant where the cells were exposed to 1 M NaC1 for twenty minutes at 22°C. Exposed films were scanned with an automated scanning densitometer (Molecular Dynamics), and the signals were expressed in relative phosphorylation units (RPU), where 1 unit is the extent of phosphorylation induced by the hypertonic stimulation.

$^{125}$I-Labeling of Stimulating Factor

The isolated *neu* receptor stimulating factor of this invention (1 μg) was radiolabelled with the amino group specific Bolton-Hunter reagent (Amersham, Arlington Heights, IL) according to published procedures (Bolton, A.E. *and* Hunter, W.M., *Biochem. J.* 133:524-538, 1973). The stimulating factor was then separated from the free reagent by gel filtration chromatography. The specific activity obtained was $8 \times 10^3$ cpm/ng.

Lipid Visualization

A modified "Oil Red O in Propylene Glycol" method was used to visualize neutral lipids, in accordance with the description in Bacus, S. et al., *Molecular Carcinogenesis* 3:350-362 (1990).

Immunocytochemical Staining

The presence of casein was detected by immunocytochemical staining with a mouse monoclonal antibody to human β and χ casein. After the medium was removed and the plastic component detached, the Lab-Tek slides were rinsed with PBS, and the cells were fixed in ethanol - formaldehyde solution (1:1, v:v) at room temperature for ten minutes. After blocking nonspecific antibody binding with 20% goat serum at room temperature, the cells were incubated with the anti-casein (β and χ) antibody (1:250 dilution) at room temperature. The slides were then rinsed with 0.5 M Tris-buffered saline (TBS), pH 7.6, and the linking antibody, biotinylated goat anti-mouse IgG (Jackson Labs, West Grove, PA), was applied to the cells at a 1:200 dilution. The cells were rinsed again with TBS, and streptavidin conjugated alkaline phosphatase (Jackson Labs, West Grove, Pa), at a 1:200 dilution, was applied to the cells. The cells were rinsed again with TBS and incubated for fifteen minutes with CAS Red and counter stained with CAS DNA stain (Cell Analysis Systems, Inc., Elmhurst, IL).

Cells were immunocytochemically stained for *neu* receptor with the HER-2/*neu* Oncogene Staining Kit (Cell Analysis Systems, Elmhurst, IL) after being fixed for thirty minutes in periodate/lysine/ paraformaldehyde (1:1:1 volume ratio) and for sixty minutes in 10% neutralized formalin.

Example 1. Purification of *neu* Receptor Stimulating Factor From Rat1-EJ 1-1 Conditioned Medium

For purification of the *neu* receptor stimulating factor, 120 liters of Rat1-EJ 1-1 conditioned media containing 9.4 grams of protein were prepared from 500 roller bottles according to the described methods. The medium was cleared by filtration through 0.2 micron filters and concentrated 31-fold with a Pelicon ultrafiltration system using membranes with a 20,000 dalton molecular weight cutoff. All subsequent protein purification steps were performed using a Pharmacia FPLC system.

Initial fractionation of the concentrated conditioned media was based on the moderate affinity of the stimulating factor to heparin (Fig. 1). The concentrated material was allowed to flow directly through a column containing 150 ml of Heparin-Sepharose (Pharmacia) that had been pre-equilibrated with PBS. The column was washed with PBS containing 0.2 M NaC1 until no decrease in absorbance at 280 nm could be recorded. The bound proteins were eluted with a 250 ml 0.2 M to 1.0 M NaC1 gradient (indicated by a dashed line in Fig. 1). Five-milliliter fractions were collected. The elution phase of this chromatographic step is depicted in Fig. 1. Samples (0.1 ml) tested for the presence of stimulating factor activity. In particular, living MDA-MB-453 cells which overexpress *neu* receptor were incubated with dialyzed samples of column fractions and the level of tyrosine phosphorylation of the receptor was measured by the use of the described tyrosine phosphorylation assay. The assay results for the indicated fraction numbers are shown as an autoradiogram (inset). Quantitative assay results (scan units) obtained from an automated densitometer scan of the autoradiogram are plotted. The closed circles on the graph of the scan data correspond to the fractions assayed in the phosphotyrosine Western blot.

Hydrophobic interaction column chromatography was used to obtain additional purification of the *neu* receptor stimulating factor (Fig. 2). Active fractions from Heparin-Sepharose column runs (total volume 360 ml) were pooled, concentrated to 25 ml by using a YM10 ultrafiltration membrane (Amicon, Danvers, MA), and ammonium sulfate was added to reach a concentration of 1.7 M. After clearance by centrifugation (10,000 x g, 15 minutes), the pooled material was loaded onto a Phenyl-Superose column (HR 10/10, Pharmacia). Column bound material was eluted by a 45 ml gradient of decreasing ammonium sulfate concentration (1.7 M to 0 mM) in 0.1 M $Na_2PO_4$, pH 7.4 (dashed line in Fig. 2). Two-milliliter fractions were collected and assayed (0.002 ml per sample) with the described *neu* receptor tyrosine phosphorylation assay (inset).

Ion exchange column chromatography was used to continue the stimulating factor purification (Fig. 3). Proteins from the Phenyl-Superose fractions 44 through 60 (0.88 mg of protein; 35 ml) were pooled, dialyzed against 50 mM sodium phosphate (pH 7.3) to remove salt, loaded onto a HR 5/5 Mono S cation exchange column (Pharmacia) that was pre-equilibrated with 50 mM sodium phosphate. The column was washed with starting buffer, then developed at a rate of 1 ml/min. with a NaCl gradient (dashed line). Column fractions were assayed at a 1:50 dilution with the described *neu* receptor tyrosine phosphorylation assay (inset). The stimulating factor activity was eluted at 0.45-0.55 M salt in fractions 22 to 25 (2 ml each).

The final column step used to obtain purified stimulating factor was metal chelate affinity column chromatography (Fig. 4). Proteins in the Mono S fractions 22 through 25 were pooled and loaded directly onto a 1 ml Hi-Trap $Cu^{2+}$ chelating Sepharose column (Pharmacia). Bound proteins were eluted with a 30-ml linear gradient of increasing ammonium chloride concentration (0-1 M; dashed line) and assayed for the induction of *neu* receptor tyrosine phosphorylation in the described assay (inset). The stimulating factor eluted in fractions 10 through 15 (0.05 to 0.2 M $NH_4Cl$), which were found to contain a single peak of protein.

This four-step column chromatography procedure yielded a purified protein with an approximate molecular weight of 44,000 daltons in a reducing SDS-PAGE gel (Fig. 5). 0.01 ml aliquots of the indicated fractions were electrophoresed in the 4-25% SDS-PAGE gel, which was then stained with a silver staining kit from ICN (Costa Mesa, CA). The presence of this protein in $Cu^{2+}$ chelating column fractions 10-15 correlates with the distribution of *neu* receptor stimulating factor in these fractions. Table I summarizes the purification procedure. Protein concentrations were determined with a protein assay kit from BioRad (Richmond, CA). Twenty micrograms of the purified protein were recovered with an overall purification of 35,115-fold and a 7.5% recovery.

Summary Of neu Receptor Stimulating Factor Purification

| Sample | Protein (mg) | Activity (RPU) | Specific Activity (RPU/mg)[*] | Purification (Fold) | Recovery (%) |
|---|---|---|---|---|---|
| Rat1-EJ 1-1 Medium | 9362 | 34546.6 | 3.69 | 1 | 100 |
| Heparin-Sepharose | 64.94 | 30715.2 | 474.8 | 128.6 | 88.9 |
| Phenyl-Superose | 2.69 | 12386.6 | 4604.6 | 1247.8 | 35.8 |
| Mono-S | 0.884 | 6248.5 | 7403.4 | 2006.3 | 18 |
| Chelating Sepharose | 0.02 | 2591.5 | 129575 | 35115 | 7.5 |

*RPU denotes relative phosphorylation units. One unit is the extent of phosphorylation obtained by using a hypertonic shock of the cells (King et al., 1990)

Example 2. Recovery of *neu* Receptor Stimulating Factor After Gel Electrophoresis.

To confirm that the *neu* receptor stimulating activity is associated with the purified protein identified in the SDS-PAGE gel in Fig. 5, two 100-ng samples of the protein purified by the method of Example 1 were electrophoresed in two lanes of a nonreducing, 4-25% SDS-PAGE gel (Fig. 6). One lane was silver stained (left panel). The other lane was cut into eight horizontal strips (labelled A-H), and the proteins were electroeluted from each strip of gel for three hours with 96 mM glycine and 10 mM Tris-HCl, pH 8.3. For electroelution, YM10 Centricons (Amicon Electroelution Apparatus) were used. The elution buffer was exchanged with PBS using the Centricon concentrators. Each eluted fraction was then assayed with the described *neu* receptor tyrosine phosphorylation assay (right panel). Quantitative assay results are presented in the center panel. The activity was recovered from gel slices that contained the approximately 44,000 dalton protein, indicating that the *neu* receptor stimulating factor activity is due to this protein and no other.

Example 3. Amino Terminal Sequence of *neu* Receptor Stimulating Factor

Although the *neu* receptor-stimulatory activity was associated with the major 44-kilodalton protein, the diffuse nature of the protein band raised the possibility that the active fraction contained more than one molecular species with this molecular weight. To test this possibility, the active fraction resulting from the last purification step (Fig. 4) was subjected to direct analysis of N-terminal amino acid sequence. A 5 µg sample (approximately 100 picomoles) of the purified material was concentrated to 0.2 ml by using an ultrafiltration cell (Amicon) and a YM10 membrane prewashed with 0.05% SDS. The N-terminal sequence analysis of the protein was performed with a Model 477 protein sequencer (Applied Biosystems, Inc., Foster City, CA) equipped with an on-line phenylthiohydantoinyl (PTH-) amino acid analyzer and a Model 900 data analysis system (Hunkapillar, M.W. et al., "Methods of Protein Microcharacterization," Shively, J.R. ed., *Humana Press*, Clifton, NJ, 1986, pages 223-247). The protein was loaded onto a trifluoroacetic acid-treated glass fiber disc precycled with polyprene and NaCl. The PTH-amino acid analysis was performed with a micro liquid chromatography system (Model 120), using dual syringe pumps and reverse-phase (C-18) narrow bore columns (Applied Biosystems, 2.1 mm x 250 mm). In one extended sequencing run, a major sequence was clearly identified through 23 cycles (Fig. 7A). Two positions were unassigned due to lack of signals. A secondary sequence (approximately one-tenth of the primary signal), corresponding to 20 amino acids starting at the third amino acid of the primary sequence (lysine), was also detectable (Fig. 7B). The recovery of amino acids in the first three cycles of protein sequencing was 85 pmoles for the major sequence. This high yield, together with the presence of an essentially single N-terminal Amino acid sequence, indicates that only one protein molecule, presumably including glycosylation variants and minor N-terminal differences, comprised the isolated active material.

Example 4. Stimulating Factor Contains Disulfide Bonds and Carbohyrates

The purified *neu* receptor stimulating factor of the present invention retained its molecular weight after reduction (Fig. 8). To directly analyze the contribution of sugars to the structure of the stimulating factor, this protein was radiolabelled with $125_I$ and subjected to enzymatic deglycosylation. Tennanogram samples of radiolabelled stimulating factor ($8 \times 10^4$ cpm) were boiled for five minutes in PBS containing 0.1% SDS. The compound 3-[3-cholamidopropyl)-dimethylammonio]-1-propanesulfonate (CHAPS) was added to 2% final concentration. 0.5 units of N-glycanase, O-glycanase and/or neuraminidase (Genzyme, Cambridge, MA) were then added and incubated with the factor for eighteen hours at 37°C. The digested proteins were separated in a 12% SDS gel. This analysis (Fig. 9) revealed that N-glycanase, which releases asparagine-linked oligosaccharides at the β-aspartylglycosylamine bond, reduced the molecular weight of the protein by only 1 to 2 kilodaltons. The effect of removal of Gal-β (1,3)-Gal NAc core disaccharides (with O-glycanase) was more extensive and led to an overall reduction of eight kilodaltons. In conclusion, O-linked sugars donate about 15% of the molecular weight of the stimulating factor, whereas N-linked sugars contribute less than 5% of the apparent mass.

Example 5. Activation of Human *neu* Receptor By Stimulating Factor

Determination of the concentration dependence of *neu* receptor tyrosine phosphorylation stimulation revealed that the *neu* receptor stimulating factor of this invention is biologically active at 1 ng/ml concentration (22 pM, Fig. 10). This concentration is similar to or even lower than the effective concentrations of other growth factors that stimulate their respective tyrosine kinases (Yarden Y. and Ullrich, A., *Ann. Rev. Biochem.* 57:443-448, 1988). Together with the amino terminal protein sequence analysis, the presented dose-dependency strongly suggests that the isolated stimulating factor of this invention is indeed the molecule responsible for *neu* receptor activation. Previously, it was not possible to separate an EGF-receptor stimulatory activity from the *neu*-specific stimulatory function. This led to the suggestion (Lupu R. et al., *Science*, 249:1552-1555, 1990) that the putative *neu* receptor stimulating factor is also a ligand or stimulating factor for the EGF-receptor. The availability of a homogeneously purified protein enabled a direct biochemical analysis of this question. Receptor specificity was examined by testing the ability of the *neu* receptor stimulating factor to stimulate tyrosine phosphorylation of the closest relative of the *neu* receptor, namely the EGF-receptor.

The results of this analysis are presented in Fig. 11. The *neu* receptor stimulating factor of this invention was able to stimulate the tyrosine phosphorylation of the *neu* receptor in MDA-MB-453 human breast carcinoma cells, but it could not activate EGF-receptor-tyrosine in human A-431 epidermoid carcinoma cells. The latter was induced by the addition of EGF, thus indicating that the specificity of the *neu* receptor stimulating factor of this invention is limited to the *neu* receptor. Nevertheless, a very small increase in EGF receptor tyrosine phosphorylation on treatment with the stimulating factor could be seen after long exposures of the film to the Western blot. However, this might have been due to an indirect transregulatory effect of the *neu* receptor stimulating factor on the EGF-receptor, in analogy to the well characterized reciprocal interaction (Stern, D.J. and Kamps, M.P., *EMBO J.* 7:995-1001, 1988; King C.R. et al., *EMBO J.* 1:1647-1651, 1988; Kokai, Y et al., *Proc. Natl. Acad. Sci. USA* 85:5389-5393, 1988).

To further exclude the possibility of direct interaction between the *neu* receptor stimulating factor and the EGF-receptor, the capacity of the isolated factor to inhibit the binding of radiolabelled EGF (10 ng/ml) to A-431 cells was tested. Whereas unlabeled EGF (100 ng/ml) inhibited 90% of $^{125}$I-EGF binding, the *neu* receptor stimulating factor of this invention at 200 ng/ml showed no competition with radiolabelled EGF, confirming its specificity to the *neu* receptor.

Example 6. Crosslinking of Stimulating Factor to *neu* Receptor.

In the foregoing Examples, the stimulating factor of this invention was purified on the basis of its ability to increase the level of *neu* receptor tyrosine phosphorylation in cells. To further confirm that the stimulatory factor acts specifically on the *neu* receptor, a method of covalent crosslinking was employed to stabilize the presumed stimulating factor receptor complex for analysis (Fig. 12).

Monolayers of cells MDA-MB-453 or SK-BR-3 (3 x 10$^5$ cells) were incubated on ice for one hour with $^{125}$I-labelled *neu* receptor stimulating factor (25 ng/ml) in the absence or presence of a 50-fold excess of unlabelled stimulating factor. The chemical crosslinker, ethylene glycolbis (succinimidyl succinate) (Pierce, Rockford, IL) was then added to a final concentration of 5 mM and incubated with the cells at 22°C for 45 minutes. After washing with PBS, the monolayers were incubated for ten minutes at 22°C with a quenching buffer (100 mM glycine in PBS, pH 7.4). Following two additional washes with PBS, cell lysates were prepared and the *neu* receptor ("*neu*-R") was immuno-precipitated with a monoclonal antibody (Ab3). An irrelevant mouse (plasmacytoma) antibody (MOPC-141, "control") was used as a control. The extensively worked immunocomplexes were separated in a 6% SDS-PAGE gel, and the dried gel was exposed at -70°C to an X-ray film with an intensifier. The procedures of immunoprecipitation and gel electrophoresis are described in Goldman, R. et al., *Biochemistry* 29:11024-11028 (1990).

The results of the analysis show that two protein bands were produced, with molecular weights of 230 (minor species) and 500 kDa (major species), respectively. Both protein complexes were immunoprecipitable with a monoclonal antibody to *neu* receptor, but not with a non-specific antibody. This result identified the 230 kDa protein as a 1:1 complex of *neu* receptor and *neu* receptor stimulating factor. The additional molecular weight band (approx. 500 kDa) most likely represents a dimer of *neu* receptor crosslinked to one or two molecules of *neu* receptor stimulating factor, similar to covalently stabilized dimers of the EGF-receptor (Cochet, C. et al., *J. Biol. Chem.* 263:3290-3295, 1988). No labeled protein was detectable in the absence of crosslinking agent ("-"), indicating the non-covalent nature of the interaction between *neu* receptor stimulating factor and *neu* receptor. In addition, a large excess (50-fold) of unlabeled stimulating factor was found to completely abolish the radioactive signal. Based on the competition by unlabelled stimulating factor, the molecular weight of the stimulating factor-containing complexes, and the recognition of the complexes by a *neu* receptor specific antibody, it can be reasonably concluded that the *neu* receptor stimulating factor of this invention is a specific binding ligand for the *neu* receptor, or interacts with a molecule that intimately associates with the *neu* receptor.

Example 7. Induction of Cellular Differentiation in Human Tumor Cells by Stimulating Factor

It has been previously observed that certain monoclonal antibodies directed to the human *neu* receptor protein induced differentiation of cells derived from a human breast carcinoma to milk-producing and growth-arrested cells (Bacus S. et al., <u>supra</u>). Therefore, the ability of the homogeneously purified *neu* receptor stimulating factor of this invention to induce phenotypic changes in cultured human breast cancer cells was tested (Table II).

## TABLE II

### Induction Of AU-565 Cell Differentiation And Inhibition Of Cell Proliferation By Stimulating Factor

| Stimulating Factor (ng/ml) | Nuclear Area ($\mu^2$) | Cell Number ($10^4/cm^2$) | Cells With Lipid droplets (%) | Cells With casein (%) |
|---|---|---|---|---|
| 0.0 | 108.0 | 8.0 | 19.0 | 24.0 |
| 0.1 | N.D. | 7.8 | 24.0 | 26.0 |
| 0.3 | N.D. | 6.8 | 35.0 | 28.0 |
| 1.0 | N.D. | 5.8 | 52.0 | 50.0 |
| 3.0 | N.D. | 5.5 | 58.0 | 80.0 |
| 6.0 | 166.0 | 4.6 | 73.0 | >90.0 |
| 10.0 | N.D. | 4.0 | 75.0 | >90.0 |
| 15.0 | 262.0 | 3.4 | 80.0 | >90.0 |

The indicated concentrations of purified stimulating factor were added to cultured AU-565 cells for four days. Determination of cell numbers and staining for lipids and casein was as described in the text. The numbers given for casein, lipids and cell density are averages obtained from 10 different microscope fields (40X magnification). The variation among fields did not exceed 15%.

EP 0 505 148 A1

For the experiments described in the Table, 0.4 x 10⁴ AU-565 cells were inoculated into Lab-Tek chamber slides (Nunc) at 0.4 x 10⁴ cells in 1-ml medium supplemented with 10% serum per chamber. Twenty-four hours later, the stimulating factor was added at the concentrations shown, and the cells were analyzed after four additional days. Cell numbers were determined by hemocytometer chamber counting or by computer-aided image analysis. The nuclear area was measured with a CAS-200 Image Analyzer after DNA staining with Fuelgen. Treatment of AU-565 cells, which overexpress *neu* receptor, with 6 ng/ml of the stimulating factor for four days, dramatically increased the fraction of cells displaying mature morphology (from 10-20% in the absence of the factor, to 90% in its presence). This phenotype included large nuclei and flat morphology and was accompanied by translocation of the *neu* receptor protein from the plasma membrane to a perinuclear localization, as revealed by immunocytochemical staining.

Since maturation of mammary epithelium ultimately leads to synthesis and secretion of milk proteins and lipids (Topper, Y.J. and Freeman, C.S., *Physiol. Rev.* 60:1049-1106, 1980), cells treated with the stimulating factor were tested for such changes. Indeed, the stimulating factor-treated AU-565 cells exhibited the appearance of large lipid droplets, which were absent or much smaller in control (untreated) cell cultures. Similarly, the appearance of casein (types $\beta$ and $\chi$) was observed in most of the the stimulating factor-treated cells (Table II). In addition to intracellular casein, intercellular staining of casein was detectable, indicating secretion of this major milk protein. Half-maximal casein and lipid effects were achieved with 40 picomolar concentrations of the stimulating factor, in agreement with concentrations needed for activation of *neu* receptor tyrosine phosphorylation (Fig. 10).

Expression of differentiation-specific genes by the mammary gland does not necessarily involve inhibition of cell growth (Schoenenberger, C.A. et al., *EMBO J.* 7:169-175, 1988; Taverna, D. et al., *Growth Diff.* 2:145-154, 1991). However, reproducible inhibition of cell proliferation was observed after treatment of human adenocarcinoma cell cultures with the purified stimulating factor of this invention (Table II). This effect of the stimulating factor was concentration-dependent, and it was apparent as soon as twenty-four hours after treatment. Analysis of DNA ploidy of the factor-treated cells revealed that growth inhibition was accompanied by a remarkable increase in DNA ploidy (Fig. 14). Incubation for 5 days with 6 ng/ml of stimulating factor yielded an overall 50% increase in the average content of cellular DNA. The largely bimodel DNA distribution reflected a large increase in the fraction of cells in the $G_2/M$ phase at the expense of $G_1/G_0$ cells. In addition to octaploidy, the stimulating factor of this invention induced even higher DNA polyploidy. These effects, together with the growth inhibition, can be interpreted as indicative for a block in the S or the $G_2/M$ phases of the cell cycle, and an uncoupling of DNA synthesis from cell division.

Practical Applications

The *neu* receptor stimulating factor of the invention is expected to be useful in treating mammalian diseases and conditions of cells expressing the *neu* receptor on their surfaces. It is expected that the factor of this invention will modulate the function of cells by stimulating and perhaps binding to the *neu* receptor on the surface of the cells, which will then alter intracellular signal transduction, leading to alteration of cell function.

For the treatment of tumors overexpressing the *neu* receptor on the surfaces of the tumor cells, the factor of the invention, an analog, a variant or an active fragment or portion thereof, is administered to the patient or to the site of the tumor where it is expected to have an effect on the tumor. Because of the factor's demonstrated utility as a growth inhibitor and as a differentiation factor for certain carcinoma cells, the factor is expected to alter properties, such as unregulated cell proliferation and undifferentiated phenotypes, of malignant tumors overexpressing *neu* receptor. Thus, the factor of this invention is expected to be useful in the therapeutic treatment of various types of tumors related to *neu* receptor expression, including breast, ovarian, prostate and gastric carcinomas.

The factor may also be combined with substances such as radiolabeled molecules, toxins, cytokines, and other compounds used for tumor treatment, in order to increase localization of these compounds on tumors expressing high levels of the *neu* receptor for the reason already explained.

Other biological or therapeutic applications are also possible. For example, normal human epithelial cells of the gastrointestinal, respiratory, urinary and reproductive tracts, as well as the skin, express the *neu* receptor on their surfaces (Press et al., *Oncogene* 5:953-962, 1990). Substances which bind to, or interact with, this receptor and alter cell growth or metabolism would be useful in situations where cell repopulation is needed, i.e., after physical injury resulting in cellular destruction, or where it is desired to increase or stimulate metabolic activities of the cell or properties resulting from cell growth and differentiation, e.g., renewed hair growth from follicles of the skin.

In other medical applications, antibodies raised against the factor of this invention, using conventional methods known to those skilled in the art, may be utilized in a diagnostic assay to detect and measure abnormal levels of the factor occurring in human disease states. For instance, such an assay can involve contacting polyclonal or monoclonal antibodies specific for the factor with a biological tissue or fluid sample under conditions appropriate for reaction of the antibodies with the factor, and then determining the level of antibody-antigen reaction (i.e., antibody binding), such as by immunochemical techniques, which is indicative of the amount of the factor in the sample. The antibodies may also be used therapeutically to affect cell proliferation as noted previously.

In another type of diagnostic assay, the stimulating factor of the invention can be used to detect a mammalian tumor expressing *neu* receptor. For example, rabiolabelled *neu* stimulating factor can be administered to patients suspected of having tumors expressing *neu* receptor. Localization of the rabiolabelled factor at the tumor site is indicative of the presence of tumor cells expressing *neu* receptor.

The factor of the invention will be formulated and dosed according to the specific disorder to be treated, the condition of the individual patient, the site of delivery of the factor, the method of administration, and other circumstances known to practitioners. Thus, for the purposes herein, an effective amount of the factor is an amount that is effective to alter cellular proliferation and differentiation, or to prevent, lessen the worsening of, alleviate or cure the condition for which the factor is administered.

The factor of the invention may be administered to the mammal in combination with a pharmaceutically acceptable carrier, such as sterile water, saline solution or other buffer, or in an emulsion. The factor of the invention may be administered to the cells of the mammal expressing the *neu* receptor on their surfaces by any convenient route, such as oral, intravenous, subcutaneous, topical, and other modes of administration. The factor of the invention is administered to the mammalian subject to be treated at a concentration and for a length of time sufficient to modulate the proliferation and differentiation of the cells. The particular concentration given will depend on such factors as the condition for which it is given, the age and weight of the recipient and the mode of administration.

The features disclosed in the foregoing description, in the following claims and/or in the accompanying drawings may, both separately and in any combination thereof, be material for realising the invention in diverse forms thereof.

## Claims

1. Purified mammalian *neu* receptor stimulating factor.

2. The factor of claim 1, which has a molecular weight of about 44,000 daltons as determined by SDS-PAGE under reducing and non-reducing conditions.

3. The factor of claim 1, being a glycoprotein.

4. The factor of claim 1, which induces tyrosine phosphorylation of the *neu* receptor at concentrations of less than or equal to 22 picomolar.

5. The factor of claim 1, having the ability to modulate cellular proliferation and differentiation.

6. The factor of claim 1, having the ability to inhibit proliferation of carcinoma cells.

7. The factor of claim 6, wherein the carcinoma cells express *neu* receptor on their surfaces.

8. The factor of claim 1, which is isolated from the rat.

9. The factor of claim 1, having the amino-terminal amino acid sequence of Figure 7A.

10. The factor of claim 1, having the amino-terminal amino acid sequence of Figure 7B.

11. A method of modulating cellular proliferation and differentiation, comprising contacting the cells with an effective amount of the factor of claim 1.

12. The method of claim 11 which is carried out in a mammal.

13. The method of claim 12 in which the mammal is a human.

14. A method of treating a mammalian tumor expressing *neu* receptor on the surface of the tumor cells, comprising administering to a mammal having such a tumor an amount of the factor of claim 1 effective to reduce tumor growth.

15. The method of claim 14 wherein the mammal is a human.

16. The method of claim 15, wherein the tumor is a breast, ovarian, prostate or gastric carcinoma.

17. The method of claim 16, wherein the factor of claim 1 is used in conjunction with one or more substances selected from cytotoxic molecules.

18. The method of claim 17, wherein the cytotoxic molecules are radiolabeled molecules, toxins and cytokines.

19. A method of diagnosing a mammalian tumor expressing *neu* receptor on the surface of the tumor cells, comprising contacting suspected tumor cells with the factor of claim 1 and measuring the binding activity of the *neu* factor, whereby the presence of such activity indicates the presence of tumor cells expressing the *neu* receptor.

20. An antibody specific for the factor of claim 1.

21. An assay for detecting the level of the factor of claim 1, comprising contacting antibodies specific for the factor with a biological tissue or fluid sample under conditions appropriate for reaction of the antibodies with the factor, and determining the level of antibody-antigen reaction as indicative of the amount of the factor in the sample.

22. The assay of claim 21 in which the antibodies are polyclonal.

23. The assay of claim 21 in which the antibodies are monoclonal.

24. A method of producing purified mammalian *neu* receptor stimulating factor, comprising subjecting media which has been conditioned by ras-transformed fibroblasts to at least three of the following four steps: heparin affinity chromatography, hydrophobic interaction chromatography, cation exchange chromatography and metal chelate affinity chromatography.

25. The method of claim 24 which comprises the following four steps, in series:
    a) heparin affinity chromatography;
    b) hydrophobic interaction chromatography;
    c) cation exchange chromatography, and
    d) metal chelate affinity chromatography.

26. The product produced by the process of claims 24 or 25.

27. An analog, variant or active fragment of the factor of claim 1 having substantially the same biological activity as the factor.

FIG. I

FIG. 2

FIG. 3

NH$_4$Cl ( M, --- )

1.00 0.75 0.50 0.25 0.00

Scan Units ( •—• )

1000 750 500 250 0

18 17 16 15 14 13 12 11 10 9 8

5 10 15 20 25 30 35 40 45 50 55 60 65 70 75 80

Fraction Number

FIG. 4

0.002 0.001 0.000

A 280 ( — )

FIG. 5

EP 0 505 148 A1

FIG. 6

EP 0 505 148 A1

# FIG. 7A

K/G-K-K-?-R-G-S-R-G-K-P-G-P-A-E-G-?-P-S-P-A-L-P

?-?-R-G-S-?-?-K-P-?-?-A-E-?-?-?-S/N-?-A-L

# FIG. 7B

βME/100°C     -       +

Mw kDa

— 80.0

— 44.6

— 30.5

— 27.5

— 12.8

FIG. 8

N-Glycanase:   -  +  +  -

O-Glycanase:   -  -  +  +

Neuraminidase:   -  -  +  +

— 106
— 80.0
— 55.4
— 49.5
— 36.5
— 32.5
— 27.5
— 21.5
— 18.5

FIG. 9

FIG. IO

**Cell line:** <u>**MDA 453**</u>  <u>**A431**</u>

NONE SF EGF  NONE SF EGF

Mr kDa

─205

─116.5

IP

─80

**Ab To:** **neu-R** **EGF-R**

FIG. 11

FIG. 12

FIG. 13

FIG. 14

European Patent Office

**PARTIAL EUROPEAN SEARCH REPORT**
which under Rule 45 of the European Patent Convention shall be considered, for the purposes of subsequent proceedings, as the European search report

Application number

## DOCUMENTS CONSIDERED TO BE RELEVANT

EP 92302291.7

| Category | Citation of document with indication, where appropriate, of relevant passages | Relevant to claim | CLASSIFICATION OF THE APPLICATION (Int Cl⁵) |
|---|---|---|---|
| D,X | CHEMICAL ABSTRACTS, vol. 111, no. 1, July 3, 1989, Columbus, Ohio, USA YARDEN YOSEF et al. "Experimental approaches to hypothetical hormones: detection of a candidate ligand of the neu protoonco-gene" page 80, column 2, abstract-no. 875z & Proc.Natl.Acad.Sci.USA 1989, 86(9), 3179/83 | 1-11, 19-27 | C 07 K 15/06 C 07 K 3/20 C 07 K 15/28 A 61 K 37/02 G 01 N 33/574 |
| A | CHEMICAL ABSTRACTS, vol. 113, no. 11, September 10, 1990, Columbus, Ohio, USA WADA TAKURO et al. "Inter-molecular association of the p185neu protein and EGF receptor modulates EGF receptor function." page 138, column 1, abstract-no. 91 965j | 1-11, 19-27 | |

TECHNICAL FIELDS SEARCHED (Int Cl⁵)

C 07 K 15/00
C 07 K 3/00
A 61 K 37/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10, 20-27
Claims searched incompletely: 11+, 19++
Claims not searched: 12-18+
Reason for the limitation of the search:

+ Art.52(4)EPC; method for treatment of the human or animal body by therapy

++ Art.52(4)EPC; diagnostic method practised on the human or animal body

| Place of search | Date of completion of the search | Examiner |
|---|---|---|
| VIENNA | 01-06-1992 | SCHARF |

30

European Patent
Office

**PARTIAL EUROPEAN SEARCH REPORT**

Application number
-2-
EP 92302291.7

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | & Cell 1990, 61(7), 1339-47 <br> -- <br> CHEMICAL ABSTRACTS, vol. 112, no. 19, May 7, 1990, Columbus, Ohio, USA FENDLY BRIAN M. et al. "Characterization of murine monoclonal antibodies reactive to either the human epidermal growth factor receptor or HER2/neu gene product." page 550, column 1, abstract-no. 176 642f & Cancer Res. 1990, 50(5), 1550-8 <br> ---- | 1-11, 19-27 | |
| | | | TECHNICAL FIELDS SEARCHED (Int. Cl.4) |

EPO Form 1505.3 06.78